# EUROPEAN PATENT SPECIFICATION

(11) **EP 2 496 145 B1**
(45) Date of publication and mention of the grant of the patent: **13.05.2020**
(21) Application number: 10798648.1
(22) Date of filing: 02.11.2010
(51) Int. Cl.: A61B 17/02, A61B 17/00

(54) **IRIS RETRACTOR**
IRISRETRAKTOR
RÉTRACTEUR D'IRIS

(30) Priority: 02.11.2009 US 257087 P
(43) Date of publication of application: 12.09.2012
(73) Proprietor: APX Ophthalmology Ltd., 34371 Haifa (IL)
(72) Inventor: ASSIA, Ehud, 69989 Tel Aviv (IL); ELIACHAR, Eliahu, 34371 Haifa (IL); LILACH, Nir, 30063 Kfar Yehoshua (IL)
(74) Representative: Boult Wade Tennant LLP
(86) International application number: PCT/US2010/055026
(87) International publication number: WO 2011/053945

(56) References cited:
- EP-A1- 0 769 271
- EP-A1- 1 029 508
- EP-A1- 1 161 925
- WO-A1-2008/115455
- US-A- 4 257 406
- US-A- 5 607 446
- US-A- 5 807 244

## Description

### FIELD OF THE INVENTION

The present invention relates generally to an iris retractor used in ophthalmic surgical procedures.

### BACKGROUND OF THE INVENTION

There are various ophthalmic procedures that require the dilation of the pupil. For example, a lens with a cataract is typically removed from the eye by phacoemulsification. This procedure breaks up the lens typically with an ultrasonically driven tool. The tool has an aspiration port that aspirates the broken lens material from the patient's ocular- chamber. It is desirable to extend the pupil during phacoemulsification to provide the surgeon with a wide view of the lens. One technique for extending the pupil includes pulling back or retracting the iris with what is referred to as an iris retractor, and holding the iris at its outer edges.

US-A-5607446 describes an instrument for dilating the pupil of an eye. The instrument includes a handle and a switch mounted on the handle for alternating between first and second states. A retractable dilator mechanism is attached to the switch and is extendible from the handle. The dilator mechanism selectively alternates between a retracted condition when the switch is in a first state and an expanded condition when the switch is in its second state.

US-A-4257406 describes a instrument for use in ophthalmic surgery to retract the iris and provide dilation of the pupil. The instrument includes a pair of curved retracting tips adapted to engage the iris gently and positively for expanding the pupil to permit removal of the crystalline lens without injury to the iris. The retracting tips are mounted on a pair of cross-action spring arms and the instrument is designed for easy comfortable one-hand operation.

WO2008/115455 describes a ring that can maintain a pupil in an extended position during an ophthalmic procedure. The ring has a plurality of loops that capture iris tissue. The ring is configured to extend the pupil when iris tissue is inserted into each loop. An ophthalmic procedure such as phacoemulsification can then be performed on the patient. The ring has a center opening that provides a wide view of the ocular chamber during the procedure.

### SUMMARY OF THE INVENTION

The present invention provides a pair of iris retractors, in accordance with claim 1. Preferred embodiments of the invention are provided in the dependent claims.

There is thus provided in accordance with the invention a pair of iris retractors, wherein each iris retractor includes a plurality of iris grabbing hooks disposed or formed at a distal end of slender elements, and a proximal handle at a proximal end of the slender elements, wherein the slender elements spring outwards resiliently moving between retracted and expanded positions by manipulation of the slender elements, wherein in the retracted position, the hooks are close to one another and the slender elements are close to one another, and wherein in the expanded position, the hooks are separate and spaced apart from each other and distal portions of the slender elements are separate and spaced apart from each other; and
an anchor or a retaining element that anchors the retractor and applies a counter force by abutting the outside of the cornea or limbus.

In accordance with an embodiment a tip of the slender element extends from a proximal sleeve.

### BRIEF DESCRIPTION OF THE DRAWINGS

The present invention will be understood and appreciated more fully from the following detailed description taken in conjunction with the drawings in which:
Figs. 1A-1C are simplified perspective, top-view and side-view illustrations, respectively, of an iris retractor, in a non-expanded orientation, constructed in accordance with an embodiment of the present invention;
Figs. 1D-1E are simplified perspective and side-view illustrations, respectively, of the iris retractor of Figs. 1A-1C, in the non-expanded orientation placed on an eye;
Figs. 2A-2C are simplified perspective, side-view and top-view illustrations, respectively, of the iris retractor of Figs. 1A-1C, in a partially expanded orientation, in accordance with an embodiment of the present invention;
Figs. 3A-3C are simplified perspective, side-view and top-view illustrations, respectively, of the iris retractor of Figs. 1A-1C, in a fully expanded orientation, in accordance with an embodiment of the present invention;
Figs. 3D-3E are simplified side-view and perspective illustrations, respectively, of the iris retractor of Figs. 1A-1C, in the fully expanded orientation placed on the eye;
Figs. 4A-4C are simplified perspective, side-view and top-view illustrations, respectively, of an iris retractor, in a non-expanded orientation, constructed in accordance with another embodiment of the present invention;
Figs. 4D-4E are simplified perspective and side-view illustrations, respectively, of the iris retractor of Figs. 4A-4C, in the non-expanded orientation placed on an eye;
Figs. 5A-5C are simplified perspective, top-view and side-view illustrations, respectively, of the iris retractor of Figs. 4A-4C, in an expanded orientation, in accordance with an embodiment of the present invention;
Figs. 5D-5E are simplified side-view and perspective illustrations, respectively, of the iris retractor of Figs. 4A-4C, in the expanded orientation placed on the eye;
Fig. 5F is a simplified perspective illustration of a modified version of the iris retractor of Figs. 4A-4C, in accordance with an embodiment of the present invention;
Figs. 6A-6C are simplified perspective, side-view and top-view illustrations, respectively, of an iris retractor, in a non-expanded orientation, constructed in accordance with yet another embodiment of the present invention;
Figs. 6D-6E are simplified side-view and perspective illustrations, respectively, of the iris retractor of Figs. 6A-6C, in the non-expanded orientation placed on an eye;
Figs. 7A-7C are simplified perspective, top-view and side-view illustrations, respectively, of the iris retractor of Figs. 6A-6C, in an expanded orientation, in accordance with an embodiment of the present invention;
Figs. 7D-7E are simplified side-view and perspective illustrations, respectively, of the iris retractor of Figs. 7A-7C, in the expanded orientation placed on the eye;
Figs. 8 and 9 are simplified perspective illustrations of different tips for the iris retractor of any of the above embodiments, in accordance with different embodiments of the present invention;
Fig. 9A is a simplified perspective illustration of the iris retractor with the distal extension of Fig. 8 or 9 in use;
Figs. 10A-10E are simplified perspective illustrations of a retractable tip for the iris retractor of any of the above embodiments, in accordance with an embodiment of the present invention, shown gradually from fully extended to fully retracted positions;
Fig. 11 is a simplified pictorial illustration of an iris retractor, constructed in accordance with another embodiment of the present invention;
Figs. 12A-12D are simplified pictorial illustrations of an iris retractor, constructed in accordance with yet another embodiment of the present invention;
Figs. 13A-13H are simplified pictorial illustrations of a manipulator for operating the iris retractor of Figs. 12A-12D, constructed in accordance with an embodiment of the present invention;
Figs. 14A-14D are simplified pictorial illustrations of an iris retractor, constructed in accordance with still another embodiment of the present invention; and
Figs. 15A-15E are simplified pictorial illustrations of an iris retractor, constructed in accordance with another embodiment of the present invention.

### DETAILED DESCRIPTION OF EMBODIMENTS

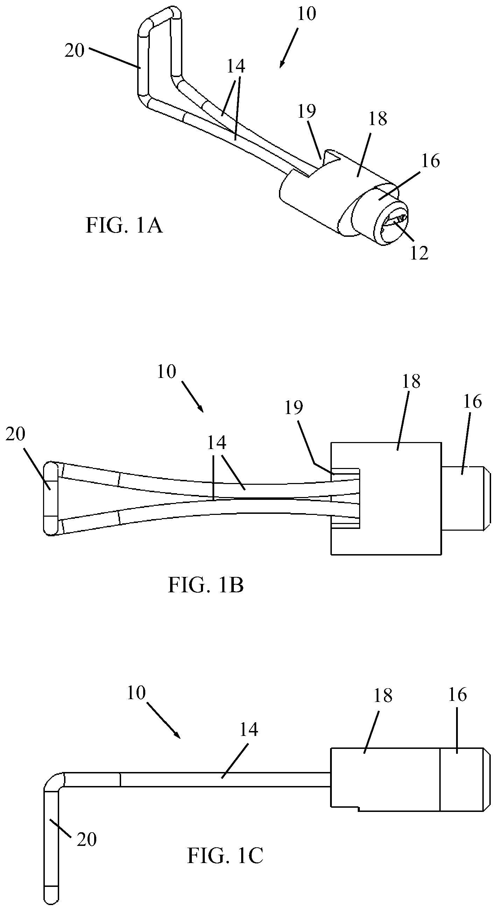

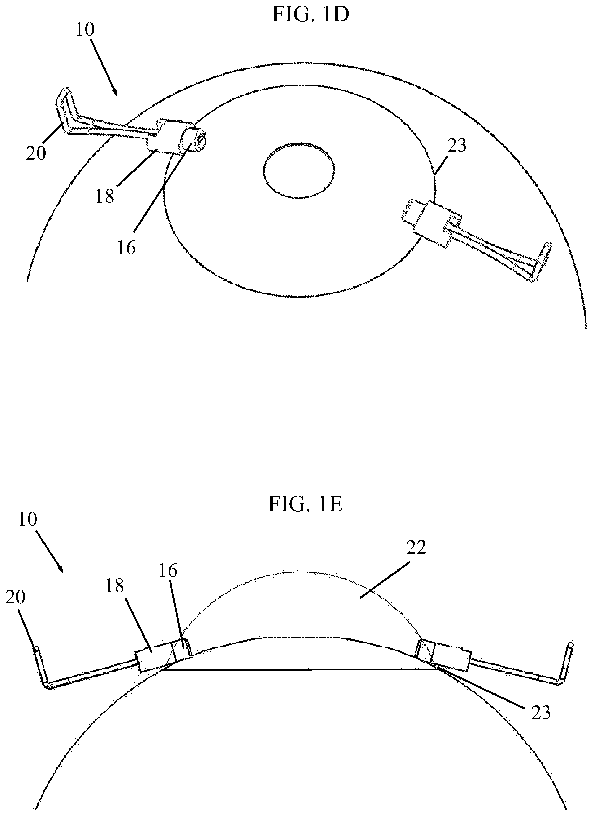

Reference is now made to Figs. 1A-3C, which illustrate an iris retractor 10, constructed in accordance with a non-limiting embodiment of the present invention.

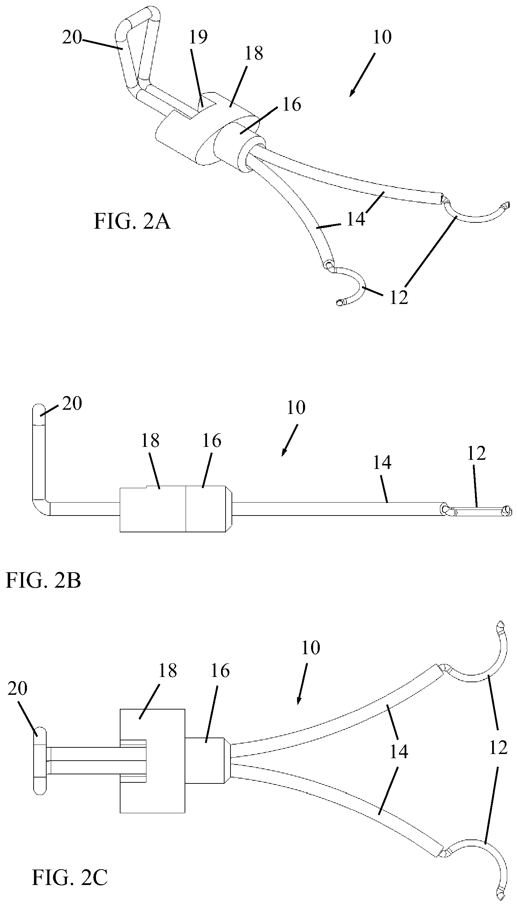

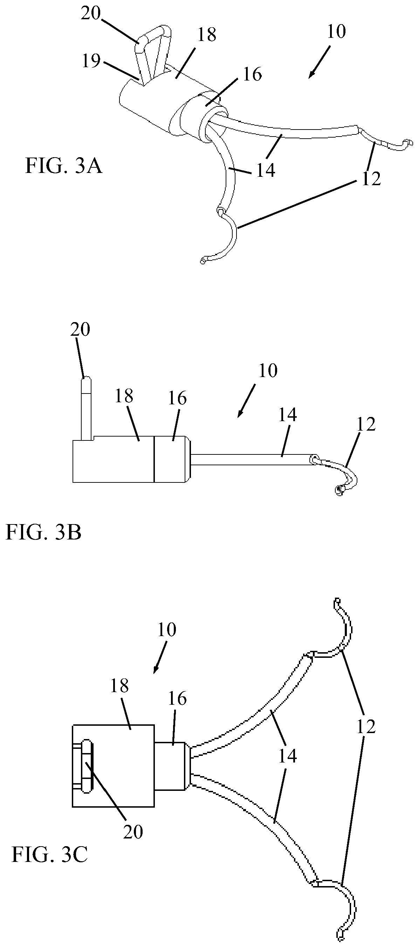

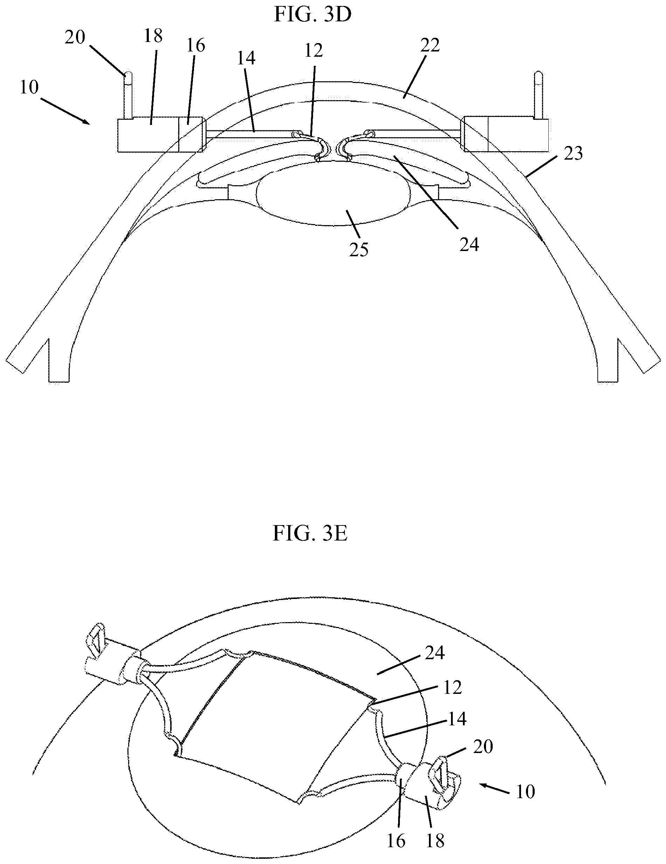

Iris retractor 10 includes a plurality of iris grabbing hooks 12 (Figs. 2A-3C) disposed or formed at a distal end of one or more slender elements 14. In the illustrated embodiment, there are two slender elements 14. The slender elements 14 are arranged to move through a retaining element 16 from a fully retracted position (Figs. 1A-1C) to a partially expanded position (Figs. 2A-2C) to a fully expanded position (Figs. 3A-3C). A proximal portion 18 of retaining element 16 is formed with a groove 19. The proximal ends of slender elements 14 terminate in a proximal handle 20. The slender elements 14 may be joined as a single element before connection to handle 20 or may be joined at the handle 20. In the fully expanded position, handle 20 is pushed completely into groove 19 and is squeezed and held in this position by the side walls of groove 19. (Alternatively, handle 20 may "click" into groove 19. Accordingly, there can be a fixed configuration, wherein handle 20 clicks into groove 19 and slender elements 14 have a fixed expansion, or an adjustable expansion configuration, wherein the more the slender elements 14 are inserted into the eye the larger is their lateral expansion.) Retaining element 16 retains slender elements 14 in the retracted position until handle 20 is pushed towards groove 19.

Slender elements 14 and hooks 12 may be constructed of a metal or plastic wire, such as but not limited, NITINOL or stainless steel or a medically safe plastic with suitable resilience, e.g., a shape memory polymer plastic.

Figs. 1D-1E illustrate a pair of iris retractors 10 in a non-expanded orientation (i.e., retracted position) placed on an eye. A portion of retaining element 16 abuts against the cornea 22, typically but not necessarily at the limbus 23. As seen in the figures, iris retractor 10 is inserted through a small incision (e.g., 1.0-1.5 mm incision) at the limbus 23. Retaining element 16 prevents iris retractor 10 from encroaching too much into the cornea 22.

Pushing handle 20 towards retaining element 16 deploys slender elements 14 and hooks 12 out of retaining element 16. As seen in Figs. 3D-3E, hooks 12 grab and hook onto the iris 24 and retract the iris 24 for exposing the lens 25 to provide a good working opening for the surgeon. Retaining element 16 anchors the retractor 10 by applying a counter force on the outside of the limbus 23.

Hooks 12 are separate and spaced apart from each other upon distal movement of slender elements 14 through retaining element 16. Thus, a single iris retractor provides spaced apart retraction points, as opposed to some prior art iris retractors which only work at a single point.

The incision for insertion of the iris retractor may be made at a different position (e.g., perpendicular thereto) than the incision made for phacoemulsification. This is advantageous because in this manner the iris retractor does not get in the way of the surgeon.

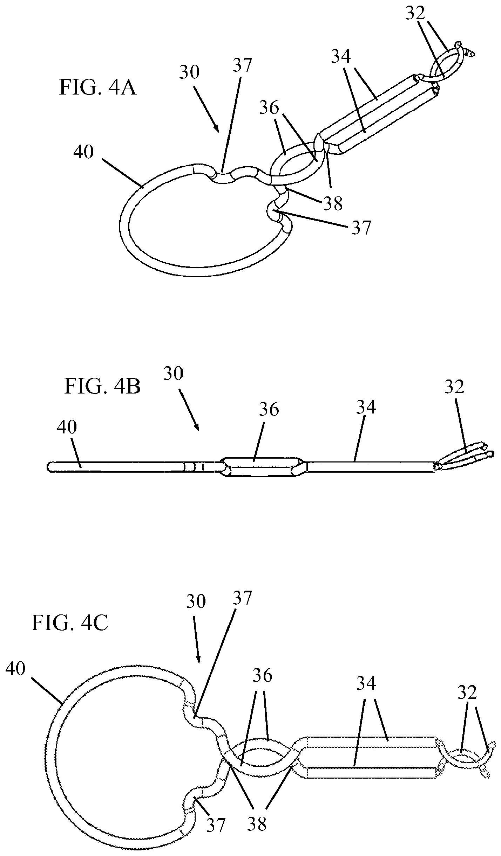

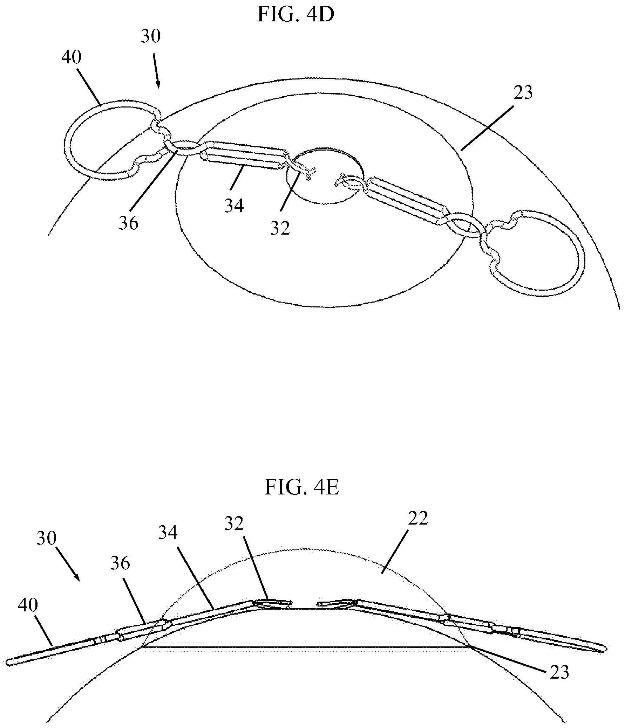

Reference is now made to Figs. 4A-5E, which illustrate an iris retractor 30, constructed in accordance with another embodiment of the present invention.

Iris retractor 30 includes a plurality of hooks 32 disposed or formed at a distal end of one or more slender elements 34. In the illustrated embodiment, there are two slender elements 34. The proximal ends of slender elements 34 terminate in a proximal handle 40. Handle 40 and slender elements 34 are made of a resilient, flexible material (e.g., metal or plastic) to form a kind of resilient tweezers or pliers. The slender elements 34 are held in the non-expanded (retracted) orientation by a retaining element 36 (which may be formed as a loop) of the slender elements 34 being caught in one or more proximal grooves 38 formed in the other slender element 34. Another option for keeping iris retractor 30 in its non-expanded state is by pressing elements 37, without slender elements 34 being caught in grooves 38.

Figs. 4D-4E illustrate a pair of iris retractors 30 in a non-expanded orientation (i.e., retracted position) placed on the eye. A portion of retaining element 36 abuts against the cornea 22, typically but not necessarily at the limbus 23.

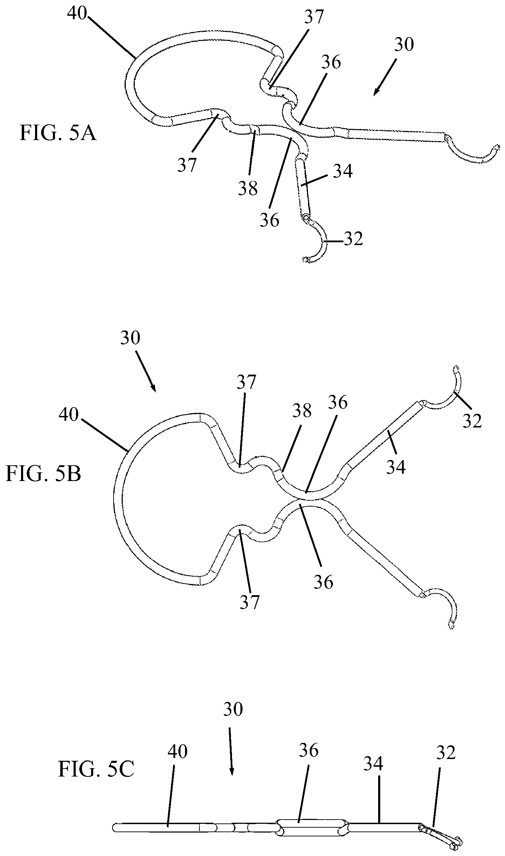

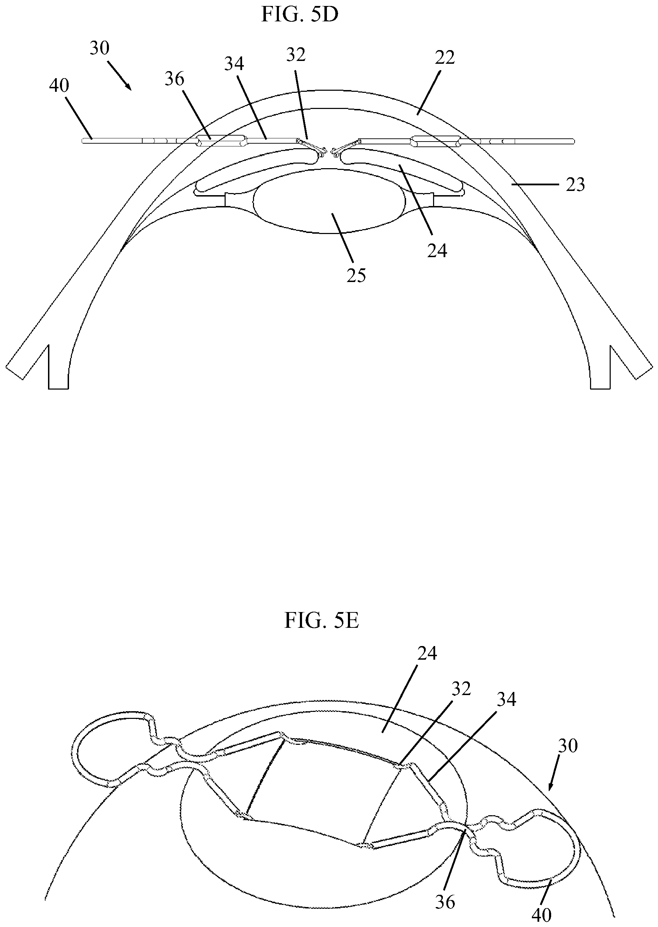

Squeezing handle 40 releases the slender element 34 that is initially caught in groove 38 of retaining element 36. (For the other option mentioned above, iris retractor 30 moves to the expanded position by releasing elements 37.) By virtue of their resilience, slender elements 34 spring outwards to the expanded position in Figs. 5A-5E. As seen in Figs. 4A-5E, the geometry of iris retractor 30 enables expansion of hooks 32 without resulting in significant expansion in the area of retaining elements 36.

As seen in Figs. 5D-5E, hooks 32 grab and hook onto the iris 24 and retract the iris 24 for exposing the lens 25 to provide a good working opening for the surgeon. Retaining element 36 anchors the retractor 30 by applying a counter force on the outside of the limbus 23.

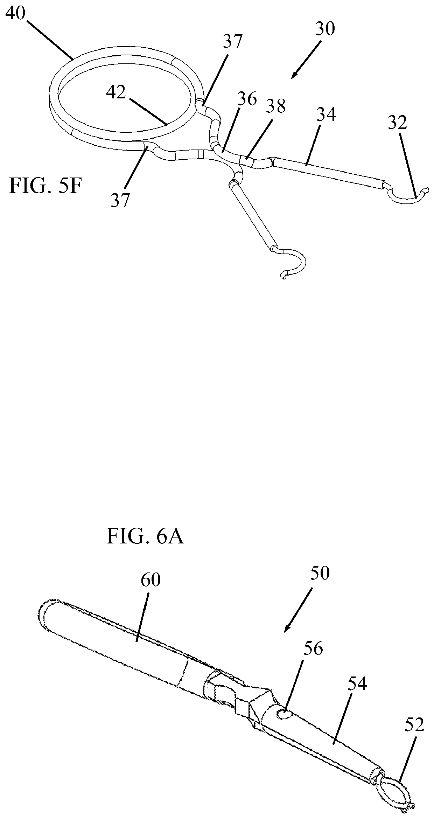

Reference is now made to Fig. 5F, which illustrates a modified version of the iris retractor 30, in accordance with an embodiment of the present invention. In this embodiment, iris retractor 30 is provided with a flexible clip 42 in handle 40. This design allows making the retractor smaller and may provide more spring (expansion) force.

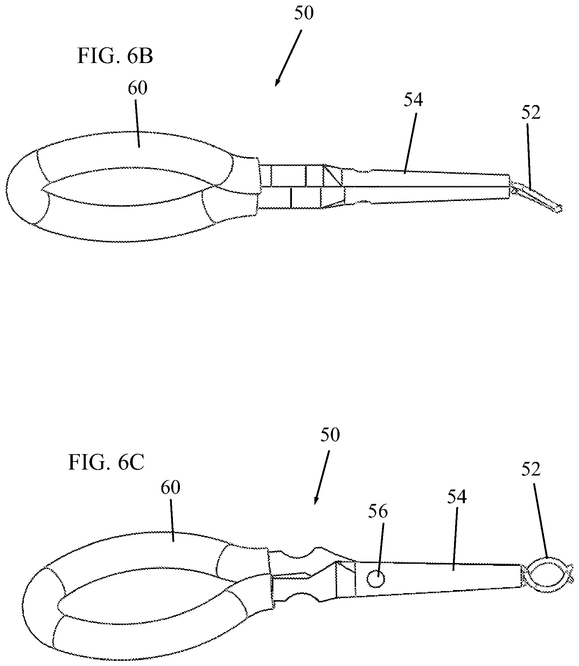

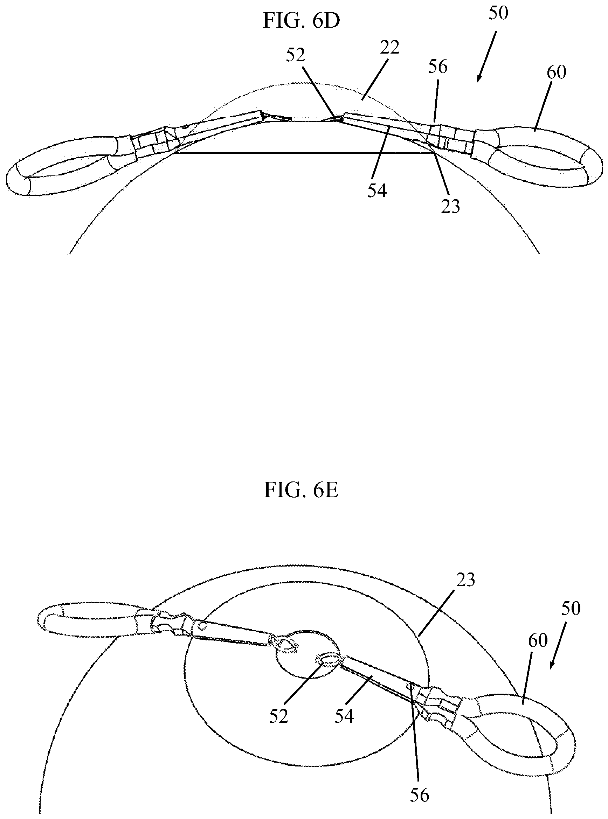

Reference is now made to Figs. 6A-7E, which illustrate an iris retractor 50, constructed in accordance with yet another embodiment of the present invention.

Iris retractor 50 includes a plurality of hooks 52 disposed or formed at a distal end of one or more slender elements 54. In the illustrated embodiment, there are two slender elements 54, which pivot about a pivot 56. The proximal ends of slender elements 54 terminate in a proximal handle 60. Handle 60, pivot 56 and slender elements 54 form a kind of scissors. Iris retractor 50 is normally expanded and slender elements 54 are held in the non-expanded (retracted) orientation by the resilience of handle 60 (thus handle 60 serves as the retaining element for initially holding the slender elements 54 in the retracted orientation.

Figs. 6D-6E illustrate a pair of iris retractors 50 in a non-expanded orientation (i.e., retracted position) placed on the eye. A portion of iris retractor 50 (e.g., near the pivot 56) abuts against the cornea 22, typically but not necessarily at the limbus 23.

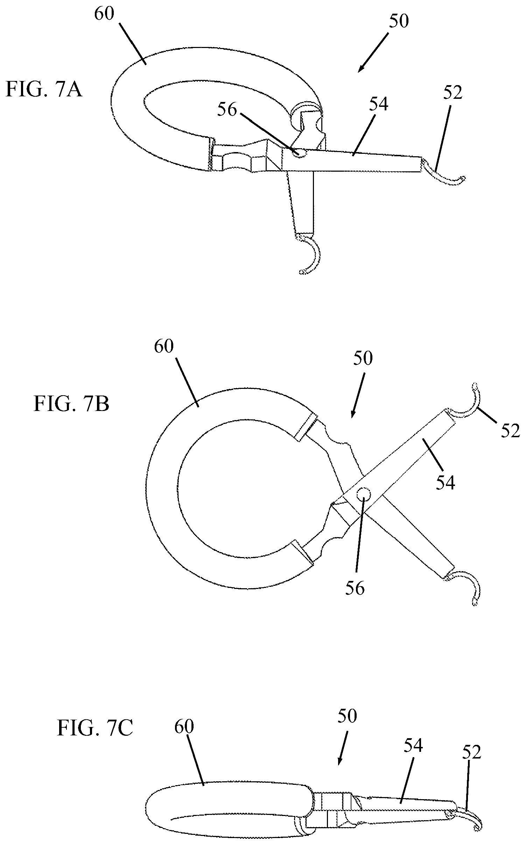

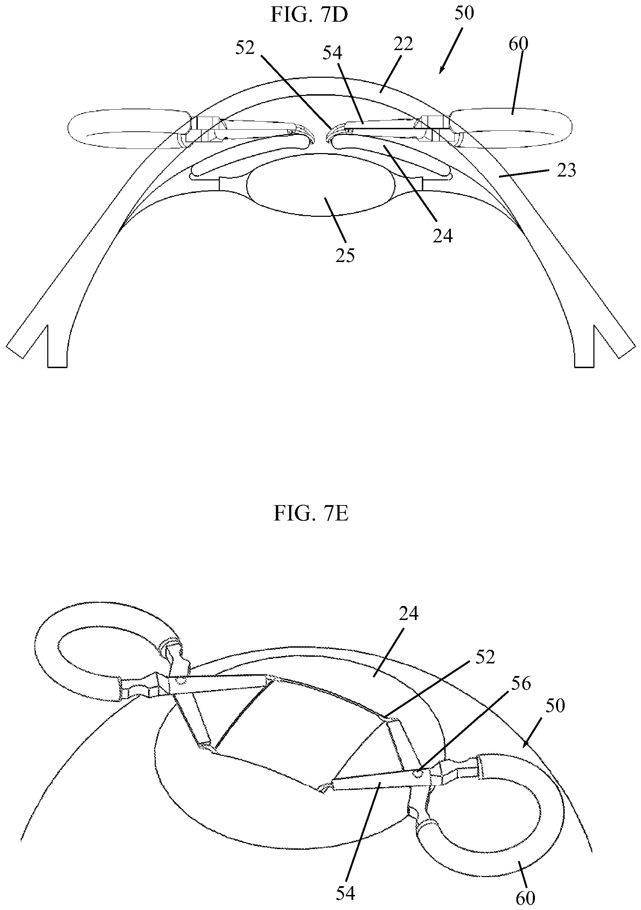

Manipulating handle 60 "scissors out" the slender elements 54 to the expanded position in Figs. 7A-7E. As seen in Figs. 7D-7E, hooks 52 grab and hook onto the iris 24 and retract the iris 24 for exposing the lens 25 to provide a good working opening for the surgeon. A portion of iris retractor 50 (e.g., near the pivot 56) anchors the retractor 50 by applying a counter force on the outside of the limbus 23.

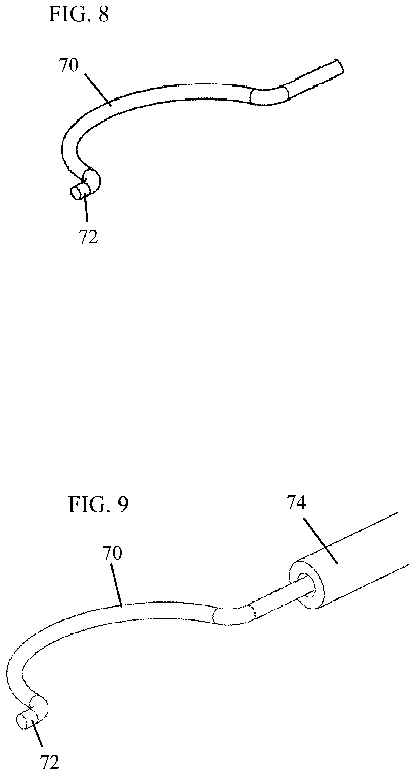

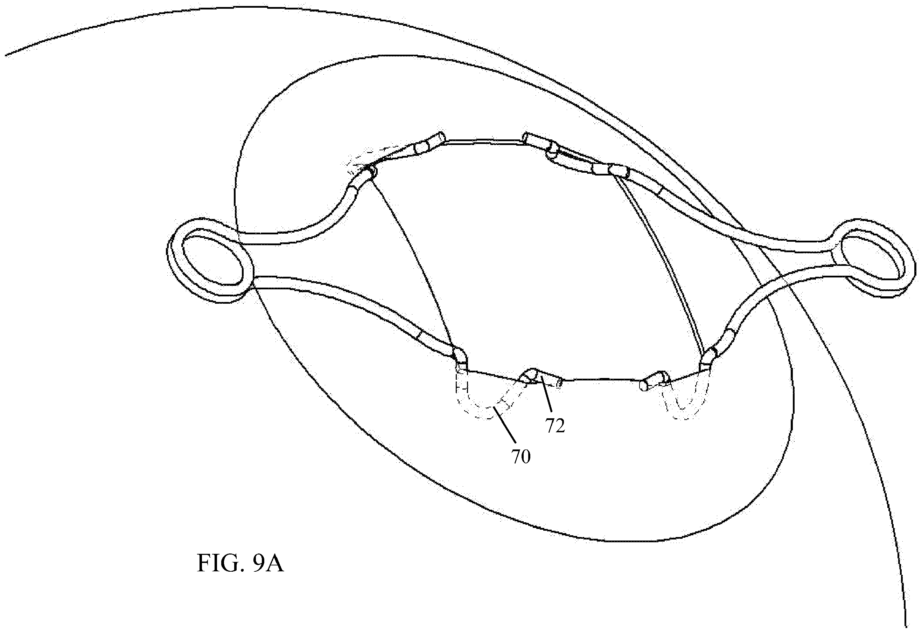

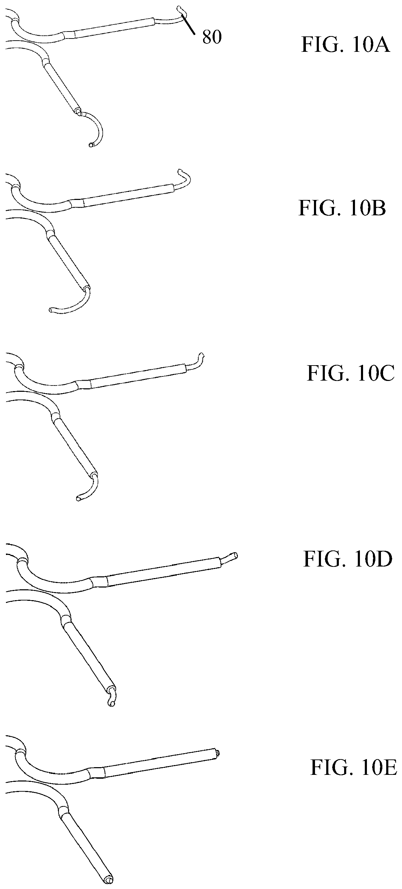

Reference is now made to Figs. 8 and 9, which illustrate different tips for the iris retractor of any of the above embodiments, in accordance with different embodiments of the present invention. In Fig. 8, a tip 70 is shown that has a U-shaped hook with a short distal extension 72. In Fig. 9, the same tip 70 is shown extending from a proximal sleeve 74. The sleeved hooks (as shown in Fig. 9) can be retracted as shown in Fig. 10.

Reference is now made to Fig. 9A, which illustrates the iris retractor with the distal extension 72 of Fig. 8 or 9 in use, in accordance with an embodiment of the present invention. It is seen that distal extension 72 firmly and positively sets the tool against the edges of the iris, and thus helps ensure proper, reliable and safe retraction of the iris.

Reference is now made to Figs. 10A-10E, which illustrate a sleeved hook 80 for the iris retractor of any of the above embodiments, in accordance with an embodiment of the present invention, shown gradually from fully extended to fully retracted positions. Sleeved hook 80 is similar to the hook shown on Fig. 9, and may or may not have a distal extension like the embodiment of Fig. 9. Any suitable retracting mechanism (not shown) may be used to retract and/or extend retractable hook 80 into and/or out of the slender elements.

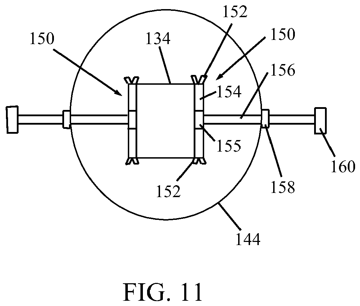

Reference is now made to Fig. 11, which illustrates an iris retractor 150, constructed in accordance with another embodiment of the present invention.

Iris retractor 150 includes a plurality of hooks 152 disposed or formed at distal ends of a first slender element 154. The first slender element 154 may be adjustable in length, such as by means of a flexible and extendable member 155 at a central portion thereof. A second slender element 156 (which may be arranged to move through a guide element, not shown, similar to that described above) is pivotally attached to first slender element 154. An anchor element 158 is mounted at a proximal position on the second slender element 156. The proximal end of second slender element 156 terminates in a proximal handle 160.

As seen in Fig. 11, the hooks 152 and first slender element 154 are inserted through a small incision at the limbus 144 and are manipulated by the surgeon so that hooks 152 spread apart and retract the iris 134. Anchor element 158 anchors the retractor by applying a counter force on the outside of limbus 144.

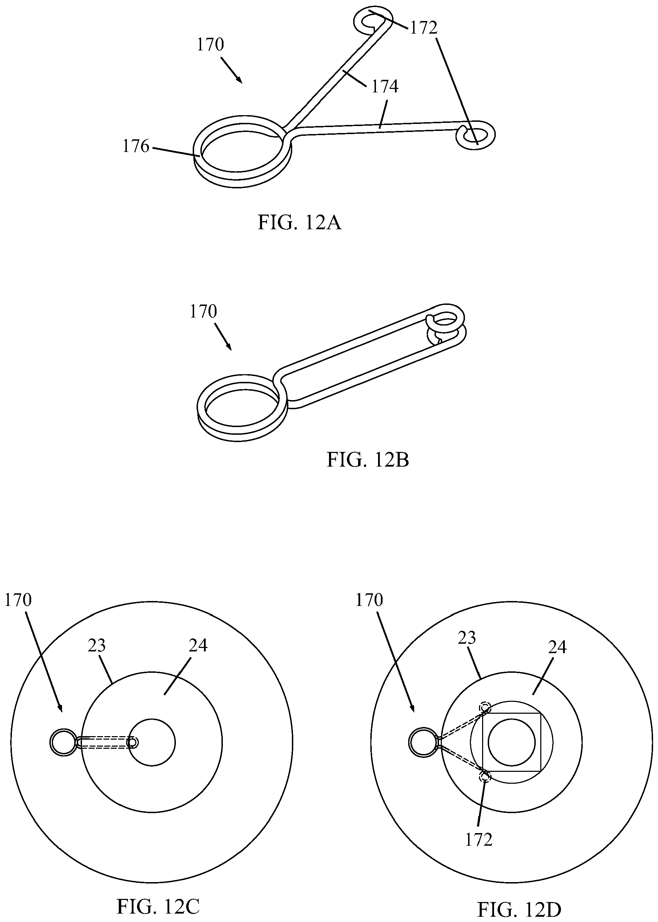

Reference is now made to Figs. 12A-12D, which illustrate an iris retractor 170, constructed in accordance with another embodiment of the present invention.

Iris retractor 170 includes a plurality of hooks 172 disposed or formed at a distal end of one or more slender elements 174. In the illustrated embodiment, there are two slender elements 174. The proximal ends of slender elements 174 terminate in a proximal handle 176. Handle 176 and slender elements 174 are made of a resilient, flexible material (e.g., metal or plastic) to form a kind of resilient tweezers or pliers. The hooks 172 in this embodiment curve back onto slender elements 174 and may optionally abut against slender elements 174.

Fig. 12C illustrates iris retractor 170 in a non-expanded orientation inserted through a small incision at the limbus 23. Fig. 12D illustrates iris retractor 170 in an expanded orientation, wherein hooks 172 grab and hook onto the iris 24 and retract the iris 24 for exposing the lens to provide a good working opening for the surgeon.

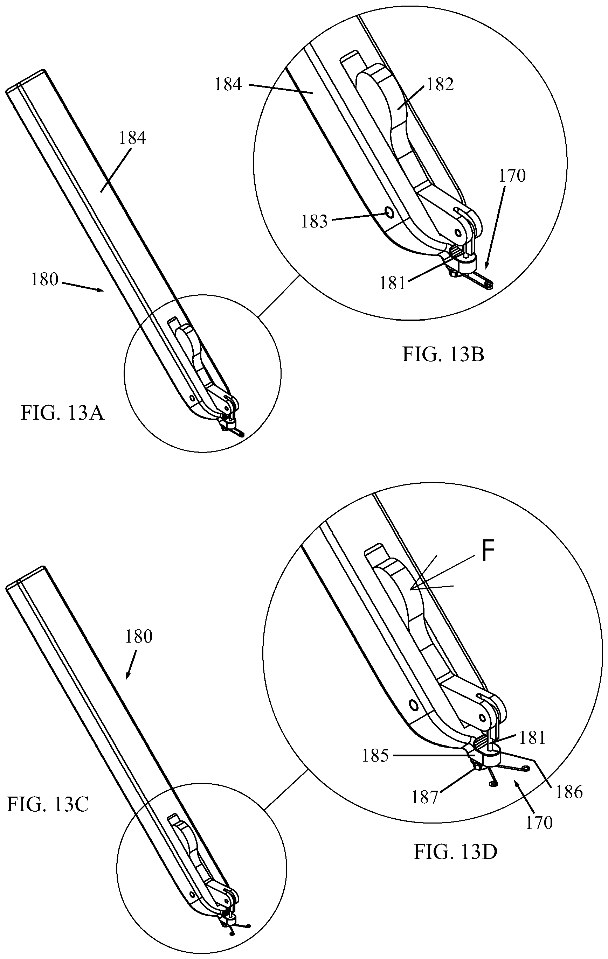

Reference is now made to Figs. 13A-13H, which illustrate a manipulator 180, for operating iris retractor 170, constructed in accordance with an embodiment of the present invention.

Manipulator 180 includes a retaining element 181 pivotally connected to a toggle lever 182, which is in turn pivotally connected at a pivot 183 on a distal end of a handle 184. The distal end of a handle 184 includes an anvil 185 formed with a hole 186 through which retaining element 181 passes. Handle 176 of iris retractor 170 fits on a lug 187 (e.g., pin) that protrudes from the bottom side of anvil 185. Lug 187 fits into the center of handle 176.

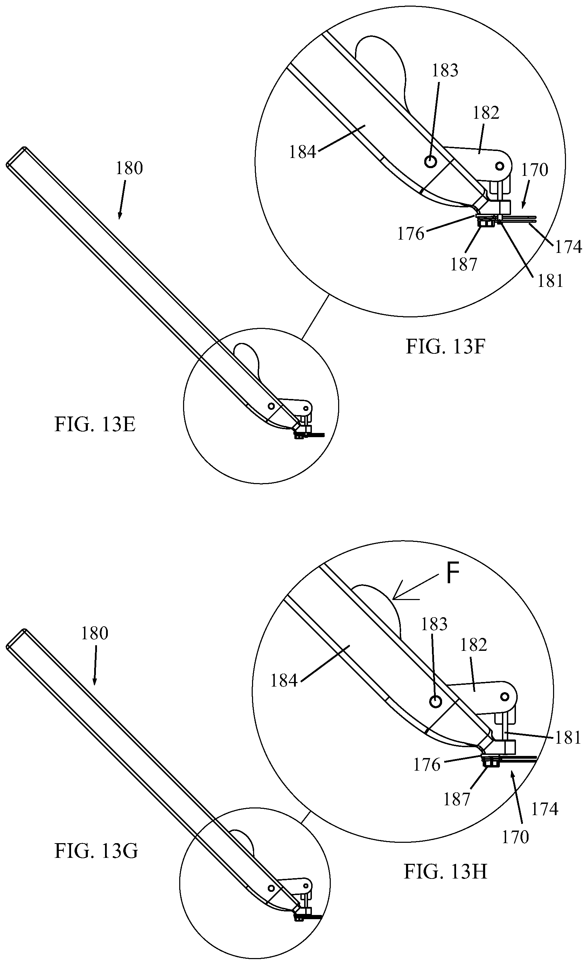

In Figs. 13A, 13B, 13E and 13F, toggle lever 182 is moved to the position wherein retaining element 181 is moved down to clamp around the slender elements 174 of iris retractor 170, thus retaining slender elements 174 in the non-expanded orientation (retracted position). In Figs. 13C, 13D, 13G and 13H, toggle lever 182 is moved to the position (indicated by arrow F) wherein retaining element 181 is moved up to release the slender elements 174 of iris retractor 170, thus allowing slender elements 174 to expand to the expanded orientation.

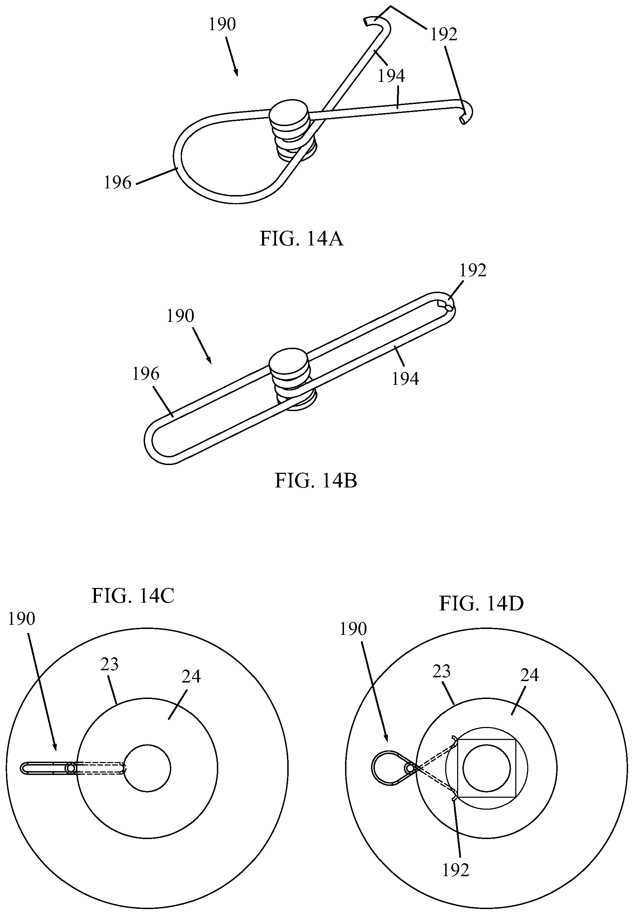

Reference is now made to Figs. 14A-14D, which illustrate an iris retractor 190, constructed in accordance with another embodiment of the present invention.

Iris retractor 190 includes a plurality of hooks 192 disposed or formed at a distal end of one or more slender elements 194. In the illustrated embodiment, there are two slender elements 194. The proximal ends of slender elements 194 terminate in a proximal handle 196. Handle 196 and slender elements 194 are made of a resilient, flexible material (e.g., metal or plastic or shape memory) to form a kind of resilient tweezers or pliers. Handle 196 in this embodiment is sufficiently resilient such that it flattens into an oblong shape when squeezed, as seen in Fig. 14B. Handle 196 springs back to its original shape to move iris retractor 190 to the expanded orientation.

Fig. 14C illustrates iris retractor 190 in a non-expanded orientation inserted through a small incision at the limbus 23. As mentioned before, handle 196 flattens into an oblong shape. Fig. 14D illustrates iris retractor 190 in an expanded orientation, wherein hooks 192 grab and hook onto the iris 24 and retract the iris 24 for exposing the lens to provide a good working opening for the surgeon.

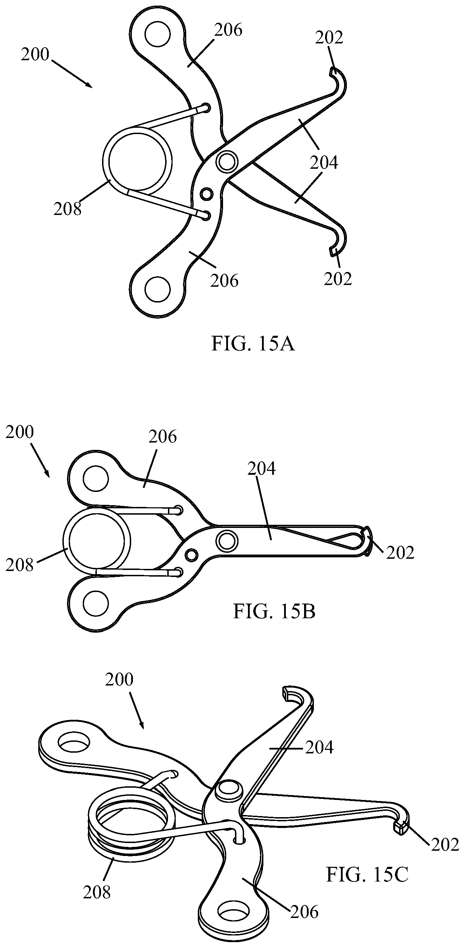

Reference is now made to Figs. 15A-15E, which illustrate an iris retractor 200, constructed in accordance with another embodiment of the present invention.

Iris retractor 200 includes a plurality of hooks 202 disposed or formed at a distal end of one or more slender elements 204. In the illustrated embodiment, there are two slender elements 204. The proximal ends of slender elements 204 form a proximal handle that includes two scissor handles 206. Handles 206 are spring loaded by a biasing device 208, such as a coil spring which has ends attached to the handles 206.

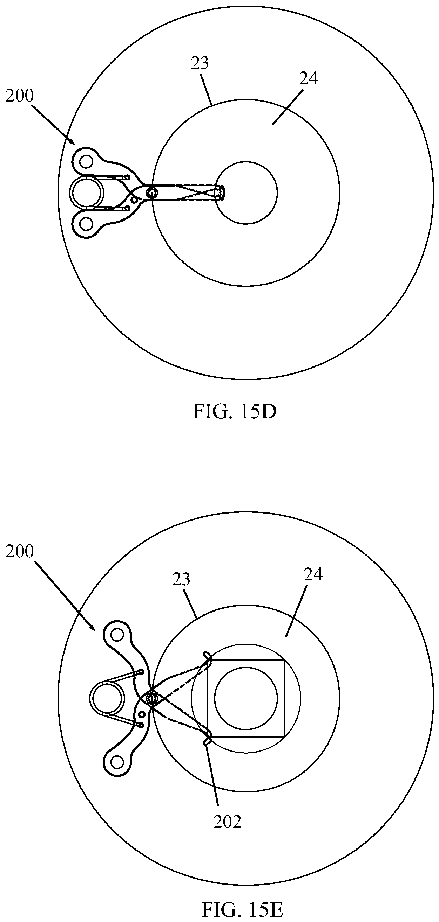

Fig. 15D illustrates iris retractor 200 in a non-expanded orientation inserted through a small incision at the limbus 23. Handles 206 are squeezed and held together so that slender elements 204 are retracted together, as shown in Fig. 15B. Fig. 15E illustrates iris retractor 200 in an expanded orientation, wherein hooks 202 grab and hook onto the iris 24 and retract the iris 24 for exposing the lens to provide a good working opening for the surgeon.

## Claims

1. A pair of iris retractors, wherein each iris retractor (10, 30, 50, 150, 170, 190, 200)
comprises:
a plurality of iris grabbing hooks (12,32,52,152,172,192,202) disposed or formed at a distal end of slender elements (14, 34, 54, 154, 174, 194, 204); and
a proximal handle (20, 40, 60, 176, 196, 206) at a proximal end of said slender elements (14, 34, 54, 154, 174, 194, 204), wherein said slender elements (14, 34, 54, 154, 174, 194, 204) spring outwards resiliently moving between retracted and expanded positions by manipulation of said slender elements (14,34,54,154,174,194,204), wherein in the retracted position, said hooks (12, 32, 52, 152, 172, 192,202) are close to one another and said slender elements (14, 34, 54, 154, 174, 194, 204) are close to one another, and wherein in the expanded position, said hooks (12, 32, 52, 152, 172, 192,202) are separate and spaced apart from each other and distal portions of said slender elements (14, 34, 54, 154, 174, 194, 204) are separate and spaced apart from each other; and
an anchor or a retaining element (16, 36, 158) that anchors the retractor and applies a counter force by abutting the outside of the cornea or limbus.

2. The pair of iris retractors according to claim 1, wherein a tip of said slender element extends from a proximal sleeve (74).

3. The pair of iris retractors according to claim 1, wherein said hooks (172) curve back onto said slender elements (174).

4. The pair of iris retractors according to claim 1, wherein said handle (196) is sufficiently resilient to flatten into an oblong shape when squeezed.

5. The pair of iris retractors according to claim 1, wherein said handle comprises two scissor handles.

## Patentansprüche

1. Paar von Irisretraktoren, wobei jeder Irisretraktor (10, 30, 50, 150, 170, 190, 200) umfasst:
eine Mehrzahl von Iris-Greifhaken (12, 32, 52, 152, 172, 192, 202), die an einem distalen Ende von schlanken Elementen (14, 34, 54, 154, 174, 194, 204) angeordnet oder ausgebildet sind; und
einen proximalen Griff (20, 40, 60, 176, 196, 206) an einem proximalen Ende der schlanken Elemente (14, 34, 54, 154, 174, 194, 204), wobei die schlanken Elemente (14, 34, 54, 154, 174, 194, 204) elastisch nach außen federn, wenn sie sich durch Manipulation der schlanken Elemente (14, 34, 54, 154, 174, 194, 204) zwischen eingezogenen und ausgefahrenen Positionen bewegen, wobei in der eingefahrenen Position die Haken (12, 32, 52, 152, 172, 192, 202) nahe beieinander liegen und die schlanken Elemente (14, 34, 54, 154, 174, 194, 204) nahe beieinander liegen, und wobei in der ausgefahrenen Position die Haken (12, 32, 52, 152, 172, 192, 202) getrennt und voneinander beabstandet sind und distale Abschnitte der schlanken Elemente (14, 34, 54, 154, 174, 194, 204) getrennt und voneinander beabstandet sind; und
einen Anker oder ein Halteelement (16, 36, 56,-158), der bzw. das den Retraktor verankert und eine Gegenkraft aufbringt, indem er bzw. sie an der Außenseite der Hornhaut oder des Limbus anschlägt.

2. Paar von Iris-Retraktoren nach Anspruch 1, bei dem eine Spitze des schlanken Elements von einer proximalen Hülse (74) aus vorspringt.

3. Paar von Iris-Retraktoren nach Anspruch 1, bei dem die Haken (172) auf die schlanken Elemente (174) zurückgebogen sind.

4. Paar Iris-Retraktoren nach Anspruch 1, bei dem der Griff (196) elastisch genug ist, um sich beim Zusammendrücken zu einer länglichen Form abzuflachen.

5. Paar von Iris-Retraktoren nach Anspruch 1, bei dem der Griff zwei Scherengriffe umfasst.

## Revendications

1. Paire de rétracteurs d'iris, dans laquelle chaque rétracteur d'iris (10, 30, 50, 150, 170, 190, 200) comprend :
une pluralité de crochets de saisie d'iris (12, 32, 52, 152, 172, 192, 202) disposés ou formés à une extrémité distale d'éléments élancés (14, 34, 54, 154, 174, 194, 204) ; et
une poignée proximale (20, 40, 60, 176, 196, 206) à une extrémité proximale desdits éléments élancés (14, 34, 54, 154, 174, 194, 204), dans laquelle lesdits éléments élancés (14, 34, 54, 154, 174, 194, 204) bondissent vers l'extérieur en se déplaçant élastiquement entre des positions rétractée et déployée par manipulation desdits éléments élancés (14, 34, 54, 154, 174, 194, 204), dans laquelle, en position rétractée, lesdits crochets (12, 32, 52, 152, 172, 192, 202) sont proches les uns des autres et lesdits éléments élancés (14, 34, 54, 154, 174, 194, 204) sont proches les uns des autres, et dans laquelle, en position déployée, lesdits crochets (12, 32, 52, 152, 172, 192, 202) sont séparés et espacés les uns des autres et des parties distales desdits éléments élancés (14, 34, 54, 154, 174, 194, 204) sont séparées et espacées les unes des autres ; et
une ancre ou un élément de retenue (16, 36, 158) qui ancre le rétracteur et applique une contre-force en venant buter contre l'extérieur de la cornée ou du limbe.

2. Paire de rétracteurs d'iris selon la revendication 1, dans laquelle une pointe dudit élément élancé s'étend depuis un manchon proximal (74).

3. Paire de rétracteurs d'iris selon la revendication 1, dans laquelle lesdits crochets (172) se recourbent sur lesdits éléments élancés (174).

4. Paire de rétracteurs d'iris selon la revendication 1, dans laquelle ladite poignée (196) est suffisamment élastique pour s'aplatir en une forme oblongue lorsqu'elle est serrée.

5. Paire de rétracteurs d'iris selon la revendication 1, dans laquelle ladite poignée comprend deux poignées en ciseaux.
